(19) Europäisches Patentamt / European Patent Office / Office européen des brevets

(11) **EP 2 813 840 B1**

(12) **FASCICULE DE BREVET EUROPEEN**

(45) Date de publication et mention
de la délivrance du brevet:
**09.10.2019 Bulletin 2019/41**

(21) Numéro de dépôt: **14305631.5**

(22) Date de dépôt: **28.04.2014**

(51) Int Cl.:
**G01N 21/65** *(2006.01)*     **G01N 33/24** *(2006.01)*
**C07C 7/12** *(2006.01)*     **B01D 15/18** *(2006.01)*
**G01N 21/84** *(2006.01)*

(54) **Dispositif et méthode d'analyse associée pour la conduite des unités de séparation de xylenes en lit mobile simulé utilisant un spectromètre Raman**

Analysevorrichtung und entsprechende Analysemethode zur Steuerung der Einheiten zur Abscheidung von Xylenen in einem simulierten Fließbett mit Hilfe eines Raman-Spektrometers

Device and related analysis method for controlling xylene separation units in simulated moving bed using a Raman spectrometer

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.06.2013 FR 1355516**

(43) Date de publication de la demande:
**17.12.2014 Bulletin 2014/51**

(73) Titulaire: **AXENS**
**92508 Rueil-Malmaison Cedex (FR)**

(72) Inventeur: **Hotier, Gérard**
**92500 Rueil Malmaison (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**US-A1- 2013 053 610     US-B2- 8 194 245**

• **MARTEAU PHILIPPE ET AL: "Remote Raman spectroscopy for process control", VIBRATIONAL SPECTROSCOPY, ELSEVIER SCIENCE, AMSTERDAM, NL, vol. 9, no. 1, mai 1995 (1995-05), pages 101-109, XP002546934, ISSN: 0924-2031**

EP 2 813 840 B1

## Description

### DOMAINE DE L'INVENTION

La présente invention se situe dans le domaine des méthodes de mesure en ligne et des dispositifs de contrôle et régulation des unités de séparation des xylènes en lit mobile simulé (désignées en abrégé par LMS) ou des unités de séparation par distillation.

Plus précisément, la présente invention concerne la mesure en ligne des compositions des flux d'hydrocarbures circulant dans les diverses zones de séparation des dites unités. Cette mesure des concentrations est obtenue à partir des spectres Raman du flux considéré, moyennant une méthode de traitement spécifique desdits spectres qui fait partie intégrante de la présente invention.

Un cas d'application particulièrement intéressant de la méthode selon la présente invention est la séparation des divers xylènes, les flux circulant dans l'unité étant constitués de mélange de meta-xylène, ortho-xylène para-xylène et d'Ethylbenzène de composition variable selon le point de mesure dans l'unité de séparation considérée.

### EXAMEN DE L'ART ANTERIEUR

Le brevet US 5,569,808 de la demanderesse décrit une méthode et un dispositif d'analyse comprenant en combinaison :

- une ou plusieurs sources émettrices laser (visible ou proche infra rouge),
- au moins 2 fibres optiques émettrices amenant ledit signal en deux points au sein de l'unité,
- de séparation de paraxylène,
- au moins deux optrodes propres à recueillir le signal Raman rétrodiffusé par 2 échantillons,
- représentatifs des flux circulant dans l'unité et à éliminer les signaux parasites,
- au moins 2 fibres optiques véhiculant les signaux recueillis par les optrodes vers un
- spectromètre comportant un détecteur comportant plusieurs plages d'analyse.

La méthode d'analyse consiste à mesurer le spectre réémis dans une gamme de longueur d'onde bien particulière ou chacun des six constituants toluène, ethylbenzène, para méta et ortho xylène, paradiethylbenzène présente un pic individuel bien distinct de ceux des 5 autres constituants.

Le document US 8194245 affine la méthode d'analyse en spécifiant notamment comment améliorer la précision de l'analyse par la prise en compte de la température de l'échantillon et comment éliminer la fluorescence de certains composés présents sous forme de traces en utilisant une ou plusieurs sources laser émettant à 785 nm.

Signalons également les brevets US 7,116,414 et US 7,548,310 qui décrivent échantillonnage et analyse RAMAN d'une manière très éloignée de la présente invention.

Le document US 2013/0053610 A1 décrit un procédé de séparation des xylènes utilisant deux colonnes d'adsorption connectées en série.

### DESCRIPTION SOMMAIRE DES FIGURES

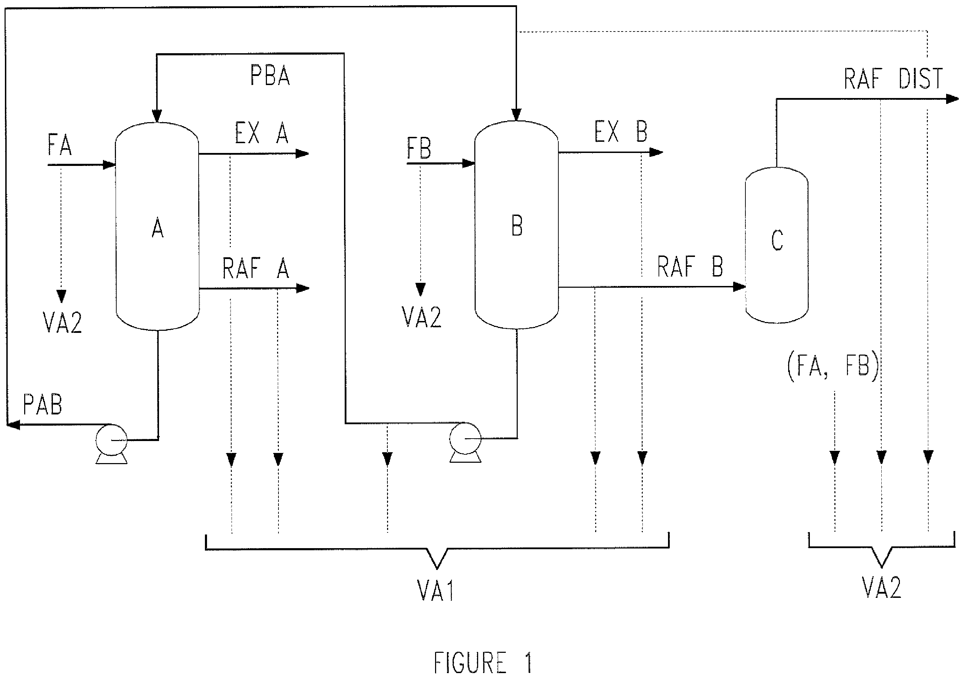

La figure 1 décrit un procédé de séparation en lit mobile simulé comprenant deux adsorbeurs A et B et les différents points d'échantillonnage qui peuvent être utilisés selon l'invention. Ces points d'échantillonnage sont les suivants :

Pour la voie d'analyse 1 (VA1) :

- liquide renvoyé de l'adsorbeur B vers l'adsorbeur A (appelé courant interne de recirculation du fond de l'adsorbeur B vers la tête de l'adsorbeur A),
- extrait issu de l'un ou l'autre des adsorbeurs,
- raffinat issu de l'un ou l'autre des adsorbeurs.

Pour la voie d'analyse 2 (VA2):

- raffinat distillé issu de la colonne de distillation C,
- charge de l'unité en entrée de l'un ou l'autre des adsorbeurs,
- liquide renvoyé de l'adsorbeur A vers l'adsorbeur B (appelé courant interne de recirculation,
- du fond de l'adsorbeur A vers la tête de l'adsorbeur B).

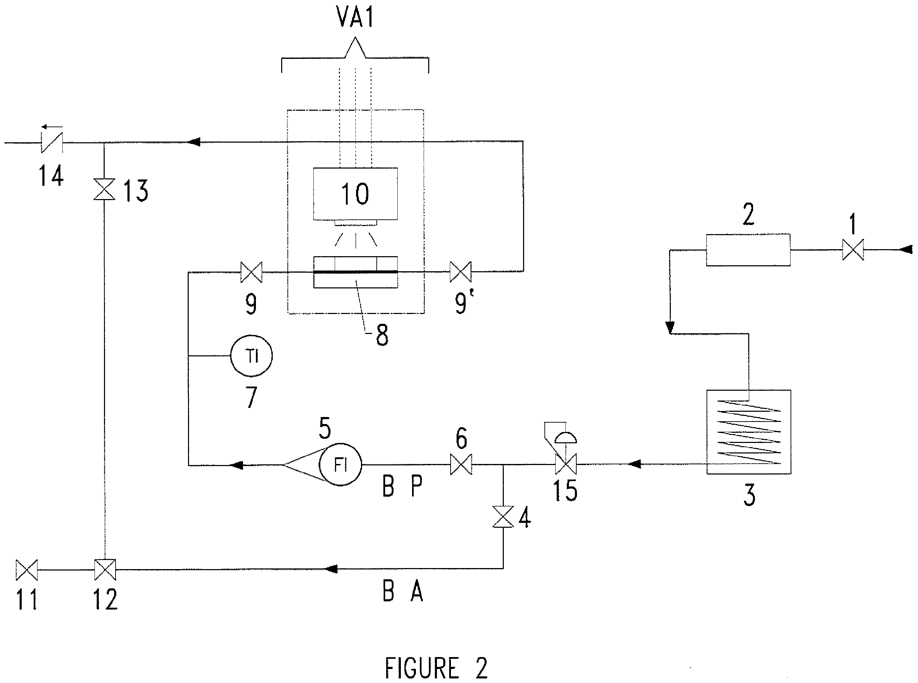

La figure 2 détaille la chaine des équipements rencontrés en suivant une voie d'analyse selon la branche principale destinée au prélèvement en vue de l'analyse Raman. Les deux voies d'analyse possédant les mêmes équipements, on a représenté seulement la voie VA1.

### DESCRIPTION SOMMAIRE DE L'INVENTION

La portée de la présente invention est définie par les revendications annexées. En particulier, la présente invention concerne une unité telle que définie dans la revendication 1 annexée et des procédés tels que définis dans les revendications 6 à 8 annexées.

La présente invention peut se définir comme un dispositif de mesure de concentration par analyse d'un spectre Raman portant sur des points de prélèvements d'une unité de séparation des xylènes en lit mobile simulé (dite unité LMS). L'utilisation de tels spectres Raman pour parvenir à une mesure des concentrations des espèces présentes dans l'unité est connue de l'art antérieur et notamment la méthodologie de passage du spectre brut aux concentrations par une méthode d'inversion de matrice décrite dans le document US 8,194,245.

Le présent dispositif s'applique à une unité de séparation des xylènes faisant appel à deux adsorbeurs A et B connectés en série, c'est à dire avec le courant de circulation issu du pied de l'adsorbeur A renvoyé en tête de l'adsorbeur B, et avec le courant de circulation issu du pied de l'adsorbeur B renvoyé en tête de l'adsorbeur A.

Plus précisément nous désignerons dans la suite l'invention comme une unité de séparation des xylènes en lit mobile simulé (dite LMS), utilisant deux adsorbeurs A et B connectés en série au sens précédent, ladite unité comportant un dispositif de mesure de concentration par analyse d'un spectre Raman portant sur des points de prélèvement, ledit dispositif étant constitué de deux voies identiques et indépendantes.

Le dispositif selon la présente invention est donc constitué de deux voies d'analyse identiques et indépendantes, dites voie 1 et voie 2, chaque voie possédant deux branches, l'une dite "principale" fonctionnant la majeure partie du temps, l'autre dite "annexe" fonctionnant une très mineure partie du temps (c'est à dire une proportion du temps inférieure à 1% sur la durée totale de fonctionnement).

Les points de prélèvements des échantillons à analyser sont définis de la manière suivante :

- pour la voie d'analyse 1 (VA1) connectée au courant de circulation interne allant de l'adsorbeur B à l'adsorbeur A :

    a) courant de circulation interne renvoyé de l'adsorbeur B vers l'adsorbeur A,
    b) extrait issu de l'un ou l'autre des adsorbeurs,
    c) raffinat issu de l'un ou l'autre des adsorbeurs.

- pour la voie d'analyse 2 (VA2) connectée au raffinat distillé issu de la colonne de distillation C :

    d) raffinat distillé (en sortie de la colonne de distillation C),
    e) charge de l'unité en entrée de l'un ou l'autre des adsorbeurs,
    f) courant de circulation interne de l'adsorbeur A vers l'adsorbeur B.

Chaque voie d'analyse (VA1) ou (VA2) comporte 2. de préférence la série d'éléments suivant pris dans le sens du flux à analyser (figure 2) :

- une vanne d'arrêt (1),
- un corps de filtre (2) dans lequel on insert une crépine filtrante de seuil de coupure compris entre 3 et 15 microns et préférentiellement compris entre 5 et 7 micromètres,

- un échangeur (3) dans lequel l'hydrocarbure circule côté tube et de l'eau de réfrigération coté calandre, échangeur comportant du coté calandre une circulation d'eau de réfrigération de température comprise entre 5 °C et 40 °C, et du coté tube une circulation d'hydrocarbures dont la température d'entrée est comprise entre 135 °C et 175 °C et la température de sortie entre 20 °C et 60 °C,
- un régulateur de pression (15) permettant de réguler la pression aval et un moyen de mesure de pression tel qu'à partir d'une pression amont comprise entre 0,9 et 1,6 MPa, on obtient une pression aval constante comprise entre 0,2 et 0,5 MPa.

Chaque voie d'analyse comporte deux branches distinctes à fonctionnement exclusif l'une de l'autre, la branche 1, dite "principale" (notée BP) étant utilisée pour l'analyse Raman et la branche 2 dite "annexe" (notée BA) étant utilisée pour des prélèvements d'échantillon à des fins d'analyse pour calibrer et/ou éventuellement recalibrer les valeurs issues de la mesure Raman.

La branche 1 de chaque voie d'analyse comporte les éléments principaux suivants pris dans le sens du flux à analyser :

- une vanne d'arrêt (6) permettant de forcer la circulation dans la branche 2 dans certaines situations,
- un thermocouple (7) permettant une prise de température de l'échantillon à analyser,
- une cellule d'analyse (8) contenant une optrode (10), recevant un signal laser d'entrée et envoyant un signal de sortie vers le spectromètre Raman, le volume interne de ladite cellule d'analyse (8) étant de l'ordre de 1 cm$^3$,
- un second ensemble de vannes d'arrêt (9, 9') pour isoler la cellule d'analyse (8) en cas de maintenance.

Selon une variante de la présente invention, le dispositif de mesure de concentration par analyse d'un spectre Raman est tel que la cellule d'analyse (8) et l'optrode (10) sont contenues dans la même cellule de confinement.

Selon une autre variante du dispositif de mesure de concentration par analyse d'un spectre Raman selon l'invention, le volume d'hydrocarbure à analyser contenu dans la cellule d'analyse (8) est renouvelé entre 2 et 25 fois par seconde.

Selon une autre variante du dispositif de mesure de concentration par analyse d'un spectre Raman selon l'invention, l'optrode (10) est reliée à une fibre optique d'entrée amenant un signal laser de longueur d'onde comprise entre 400 nm et 1080 nm, et préférentiellement comprise entre 520 nm et 785 nm (nm étant l'abréviation de nanomètre soit 10$^{-9}$ m).

Selon une variante de la méthode selon l'invention, pour la plus petite partie du temps correspondant à des situations transitoires, la voie d'analyse 2 est connectée à la charge injectée vers l'un ou l'autre des adsorbeurs.

Selon une autre variante de la méthode selon l'invention, en cas de maintenance sur la voie d'analyse 1 (VA1), la voie d'analyse 2 (VA2) est connectée au courant de circulation interne de l'adsorbeur A vers l'adsorbeur B.

Selon une autre variante de la méthode d'analyse des concentrations selon l'invention, le volume total du circuit entre le point de prélèvement et le point de mesure est tel que le temps de circulation entre lesdits points est compris entre 1 et 75 secondes, la vitesse linéaire dans la cellule d'analyse (8) étant comprise entre 0,08 et 0,12 m/s.

Selon une autre variante de la méthode d'analyse des concentrations selon l'invention, les éléments du dispositif sont calculés pour assurer la combinaison de conditions opératoires suivantes au niveau de la cellule d'analyse (8) :

1) échantillon substantiellement exempt de particules solides en suspension (c'est à dire en contenant moins de 10 ppb, ppb signifiant partie par billion)

2) échantillon dont la température est comprise entre 20 °C et 60 °C

3) échantillon dont la pression est comprise entre 0,2 et 0,5 MPa (1 MPa = $10^6$ Pa)

4) vitesse linéaire comprise entre 0,02 et 0,25 m/s et de préférence entre 0,05 et 0,2 m/s, et de manière très préférée entre 0,08 et 0,15 m/s.

Selon une autre variante de la méthode d'analyse des concentrations selon la présente invention, en cas de fonctionnement dégradé de l'unité en lit mobile simulé, on déconnecte la ligne d'échantillonnage de la première voie d'analyse (VA1) pour la connecter sur l'un des points suivants :

- Extrait en sortie d'adsorbeur A ou B (selon que A ou B présente un fonctionnement dégradé), juste en amont de la vanne de contrôle d'extrait,
le couple température pression étant choisi de la façon suivante: pression variable de 0,9 à 1,4 MPa (1 MPa = $10^6$ Pa), température comprise dans l'intervalle de 150 °C à 185 °C,
- Raffinat en sortie d'adsorbeur A ou B (selon que A ou B présente un fonctionnement dégradé): juste en amont de la vanne de contrôle de raffinat,
le couple température pression étant choisi de la façon suivante: pression variable de 0,9 à 1,4 MPa, température comprise entre 150 °C et 185 °C,

Selon une autre variante de la méthode d'analyse des concentrations selon la présente invention, la seconde voie d'analyse (VA2) s'applique à l'un des points de prélèvement suivants :

- Raffinat distillé : localisé au refoulement de la pompe de charge de l'unité d'isomérisation, température comprise entre 120 °C et 160 °C, pression comprise entre 1 et 1,6 MPa,
- Charge : localisé entre le refoulement de la pompe de charge et la vanne de contrôle de charge, température comprise entre 150 °C et 185 °C, pression comprise entre 1,6 et 2 MPa,
- Liquide de recirculation renvoyé du pied de l'adsorbeur A vers la tête de l'adsorbeur B: température comprise entre 0 °C et 80 °C, pression comprise entre 0,15 et 0,5 MPa.

Il est décrit également une méthode d'échantillonnage coordonnée au fonctionnement de l'unité en LMS, apte à délivrer autant d'échantillons (N) qu'il y a de lits (N) dans l'unité de séparation en lit mobile simulé (LMS), le début du prélèvement de chacun des échantillons N étant réalisé aux instants t1, t2, t3... tN reliés entre eux par les relations :

$$t2 = t1 + T/N$$

$$t3 = t2 + T/N$$

T désignant le temps de cycle du procédé de séparation en LMS, c'est à dire le temps au bout duquel les positions des points d'injections et de soutirage sont revenues à leur position initiale, et t1 étant un temps arbitraire choisi dans l'intervalle 5, (T/N)-5 exprimé en secondes (le chiffre 5 a donc la signification physique de 5 secondes et est homogène à T exprimé également en secondes), et la durée de chaque prélèvement θ étant comprise entre 0,5 et 50 secondes, et préférentiellement comprise entre 2 et 20 secondes.

De préférence, ladite méthode d'échantillonnage est mise en oeuvre avec le dispositif selon l'invention.

Il est décrit également un procédé d'étalonnage (ou de réétalonnage) du spectromètre Raman en liaison avec la méthode d'échantillonnage, caractérisé par les étapes suivantes:

1- on utilise les N échantillons prélevés depuis la branche annexe (BA) selon la méthode d'échantillonnage précédente,

2- on analyse précisément chacun des N échantillons par une méthode autre que la spectrométrie Raman, par exemple par chromatographie,

3- on compare les résultats de l'analyse de l'étape 2 avec ceux obtenus par le spectromètre Raman de manière à réajuster les coefficients d'étalonnage pour chacun des constituants.

## DESCRIPTION DETAILLEE DES MODES DE REALISATION PREFERES DE L'INVENTION

## 1) PRESENTATION DU DISPOSITIF SELON L'INVENTION

La présente invention peut se définir comme un dispositif de mesure de concentration par analyse d'un spectre Raman portant sur des points de prélèvements d'une unité de séparation des xylènes en lit mobile simulé (dite unité LMS). Ladite unité de séparation en LMS est constituée de deux adsorbeurs notés A et B connectés en série, c'est à dire qu'il existe un premier flux de recirculation reliant le fond de l'adsorbeur B à la tête de l'adsorbeur A et un second flux de circulation reliant le fond de l'adsorbeur A à la tête de l'adsorbeur B.

Le dispositif selon la présente invention est constitué de deux voies identiques et indépendantes, dites voie d'analyse 1 (VA1) et voie d'analyse 2 (VA2), chaque voie possédant deux branches l'une dite principale (notée BP) fonctionnant la majeure partie du temps, l'autre dite annexe (notée BA) fonctionnant une mineure partie du temps, et les points de prélèvements étant définis de la manière suivante :

-     pour la voie d'analyse 1 (VA1) connectée au courant de circulation interne allant de l'adsorbeur B à l'adsorbeur A :

      a) courant de circulation interne renvoyé de l'adsorbeur B vers l'adsorbeur A,
      b) extrait issu de l'un ou l'autre des adsorbeurs,
      c) raffinat issu de l'un ou l'autre des adsorbeurs,

-     pour la voie d'analyse 2 (VA2) connectée au raffinat distillé issu de la colonne de distillation C :

      d) raffinat distillé (en sortie de la colonne de distillation C),
      e) courant de circulation interne de l'adsorbeur A vers l'adsorbeur B,
      f) charge de l'unité en entrée de l'un ou l'autre des adsorbeurs,

chaque voie d'analyse comportant deux branches distinctes à fonctionnement exclusif l'une de l'autre, la branche 1, dite "principale" (BP) étant utilisée pour l'analyse Raman, la branche 2 dite "annexe" (BA) étant utilisée pour le prélèvement d'échantillon, et la branche 1 de chaque voie d'analyse comportant les éléments principaux suivants pris dans le sens du flux à analyser:

-     une vanne d'arrêt (6) permettant de forcer la circulation dans la branche 2 (BA) dans certaines situations,
-     un thermocouple (7) permettant une prise de température de l'échantillon à analyser,
-     une cellule d'analyse (8) contenant une optrode (10), recevant un signal laser d'entrée et envoyant un signal de sortie vers le spectromètre Raman, le volume interne de ladite cellule d'analyse (8) étant de l'ordre de 1 cm3,
-     une seconde vanne d'arrêt (9) pour isoler la cellule d'analyse (8) en cas de maintenance.
-     Le circuit se sépare en 2 branches parallèles au moyen d'un raccord en Té

Branche n°1 dite principale pour l'analyse Raman (et notée BP) :

Cette branche principale comprend les éléments suivants :

-     Une vanne d'arrêt (6) permettant de forcer la circulation dans la branche N°2 (et d'isoler la cellule à circulation),
-     Un débitmètre local (5) et une vanne pointeau manuelle permettant de régler un débit entre 5 et 60 l/h,
-     un thermocouple (7),
-     une cellule d'analyse (8) de volume interne 1cm3, telle que le liquide circule entre 2 hublots en saphir,
-     une couple de vannes d'arrêt (9, 9') pour isoler la cellule d'analyse (8) en cas de maintenance,
-     les 2 branches parallèles se réunissent au moyen d'un Té

Selon les règles de sécurité en vigueur dans les différents sites, l'ensemble constitué de l'optrode (10) et de la cellule d'analyse (8) est préférentiellement logé dans une enceinte de confinement. Dans ce cas, on trouve en outre dans la branche N°1 à l'entrée et à la sortie de la ligne de circulation dans l'enceinte un arrêteur de flamme (non représenté sur la figure 2).

Branche n°2 dite annexe pour le prélèvement d'échantillons (et notée BA) :

La circulation dans la branche N° 2 se fait de manière très exceptionnelle (typiquement moins de 1% du temps de fonctionnement).

La branche annexe (BA) comprend les éléments suivants :

-     une vanne pointeau (11) permettant de régler le débit dans la branche d'échantillonnage,
-     une vanne pneumatique 3 voies-2 positions (12), la position d'échantillonnage étant dirigée vers une vanne pointeau permettant d'ajuster exactement la quantité d'échantillon prélevée au volume des flacons disponibles.
-     une vanne d'arrêt (13) permettant de forcer la circulation dans la branche N°1 (BP)
-     un clapet anti retour (14) en sortie de la première voie d'analyse (VA1),

Un té permet de rejoindre la ligne de retour des deux voies d'analyse vers un point de plus basse pression.

Selon une variante préférée, le volume total du circuit entre le point de prélèvement et le point de mesure est tel que le délai de circulation entre les deux points est compris entre 1 seconde et 75 secondes lorsque la vitesse linéaire dans la cellule d'analyse (8) est comprise entre 0,08 et 0,12 m/seconde.

Dans l'unité de séparation de paraxylène en lit mobile simulé, on manipule généralement des liquides hautement inflammables à température et pression élevée. Les équipements doivent donc être en général antidéflagrants, et respecter les réglementations locales en matière d'équipements susceptibles de rentrer en contact avec des atmosphères explosives; (par exemple, la réglementation ATEX issue des directives européennes 94/9/CE ou ATEX 137). En particulier les alimentations électriques sont généralement en basse tension et les câbles protégés par des gaines métalliques.

Les équipements sont généralement dimensionnés pour tenir en pression et donc, soit être protégés par des soupapes de sécurité tarées pour s'ouvrir bien avant la rupture mécanique dudit équipement, soit tenir à la pression de refoulement maximum des pompes.

Les équipements de petite taille destinés à conditionner les flux analytiques sont généralement protégés par une armoire métallique fermée. Les fibres optiques véhiculant le signal laser sont généralement confinées dans des gaines métalliques.

**2) CHOIX DES FLUX A ANALYSER :**

Comme il a déjà été indiqué, en fonctionnement normal la voie d'analyse 1 (VA1) traite le courant de circulation interne renvoyé de l'adsorbeur B vers l'adsorbeur A, et la voie d'analyse 2 (VA2) traite le raffinat distillé (en sortie de la colonne de distillation C).

Néanmoins dans certaines circonstances (démarrage, situations de dysfonctionnement...), il est possible de traiter d'autres flux qui sont indiqués ci dessous.

1°) La charge à séparer contenant du paraxylène:

La plupart du temps (fonctionnement en régime établi du groupe d'unités constituant le complexe aromatique) la charge à séparer dans l'unité en LMS est de composition constante et ne dépend que des conditions opératoires et du fonctionnement des unités de réformage catalytique, de transalkylation, et d'isomérisation des C8 aromatiques.

Une analyse en temps réel et en continu n'est donc pas d'une grande utilité.

Un échantillonnage une fois par jour avec un résultat fourni quelques heures plus tard est amplement suffisant. Cependant, lors des phases transitoires (mise en route, changements de charge à l'entrée du complexe, changement de conditions opératoires de l'une des unités précitées), cette composition peut varier assez rapidement, et dans ce cas, l'analyse en temps réel et en continu de la charge permet d'anticiper les réglages à effectuer sur l'unité de séparation en lit mobile simulé.

2°) Le courant interne de recirculation:

Dans l'unité de séparation en LMS, il s'établit un chromatogramme ou profil de concentration, dont la particularité est de se déplacer en permanence autour d'une boucle constituée par les lits de tamis moléculaire disposés dans les adsorbeurs. Ce profil fait de 1, 2 à 3 tours de boucle à l'heure. A l'intérieur du profil on observe des variations de forte pente et amplitude des concentrations en constituants (à une échelle de l'ordre de la minute).

La forme de ce profil de concentration est, pour une température constante et une charge de composition donnée, uniquement fonction des 4 débits internes de liquide, et du temps de permutation des lits de solide adsorbant.

Au quotidien l'exploitant de l'unité doit pouvoir procéder à 2 types de réglages :

- augmentation ou diminution de la production pour s'adapter à la demande et
- réglage du couple pureté rendement en fonction des toutes petites variations de température
- ou de composition de charge, ou encore de dérive des contrôleurs de débit.

Le courant interne de recirculation doit donc, selon l'invention, être analysé en permanence en au moins un point, et dans certains cas analysé en 2 points. En fonctionnement normal, le courant de recirculation du pied de l'adsorbeur B vers la tête de l'adsorbeur A est analysé au moyen de la voie d'analyse 1 (VA1).

3°) Le raffinat après distillation (charge de l'unité d'isomérisation située en aval) :

La mesure en continu de la teneur résiduelle en paraxylène de ce flux donne la perte (ou par différence le rendement) de l'unité de séparation en lit mobile simulé. La précision de l'analyse est suffisante pour mesurer des teneurs en paraxylène généralement comprises entre 0,1% et 2% poids, et de manière préférée entre 0,2% et 0,9% poids.

Cette information couplée à la précédente permet de régler l'unité au quotidien. Il est très difficile de mesurer la pureté du paraxylène après distillation. L'analyseur Raman n'est généralement pas assez précis pour mesurer des traces de métaxylène, orthoxylène et ethylbenzène de l'ordre de 0,04 à 0,1% poids pour chacun de ces trois composés.

4°) L'extrait et/ou le raffinat :

En cas de dysfonctionnement de l'unité de séparation en LMS, il peut éventuellement être intéressant de mesurer la composition de l'extrait et/ou du raffinat directement en sortie des lits de tamis moléculaire afin de détecter une anomalie reliée à un lit particulier.

Le procédé d'échantillonnage selon la présente invention est caractérisé par le fait que les éléments du dispositif sont calculés pour assurer simultanément :

1) la coordination avec les permutations des N lits constituants l'unité de séparation en LMS,
2) une vitesse linéaire de l'échantillon dans la ligne comprise entre 0,02 et 0,25 m/sec, et de préférence entre 0,05 et 0,2 m/sec, et de manière très préférée entre 0,08 et 0,12 m/sec.

## 3) LOCALISATION DES POINTS D'ECHANTILLONNAGE :

Le but est de minimiser le trajet entre les points d'échantillonnage et l'optrode (10) d'une part, et entre les points d'échantillonnage et le point de collecte des flux analysés, d'autre part.

Cette disposition en trajet minimal permet d'établir une boucle d'échantillonnage rapide.

Les flux du procédé industriel de séparation par adsorption circulent à température (de 140 à 210 °C) et pression (de 0,1 MPa à 2 MPa relatif) assez élevées et dans des canalisations de diamètre généralement compris entre 0,1 à 0,5 m.

On réalise, sur la grosse canalisation, un piquage de diamètre beaucoup plus faible (typiquement de 0,003 à 0,012 m) en un point choisi pour avoir la plus forte pression disponible.

Pour la première voie d'analyse (VA1), le piquage est de préférence localisé entre l'une des pompes de recirculation et une vanne de contrôle des débits internes ou de la pression de recirculation.

Le couple température pression est choisi de la façon suivante: 150 °C à 185 °C, 1,6 à 2 MPa.

La pression maximum de refoulement de la pompe à débit nul : 4 MPa.

En cas de fonctionnement dégradé de l'unité en lit mobile simulé on déconnecte la ligne d'échantillonnage de la voie d'analyse 1 (VA1) pour la connecter sur l'un des 2 points suivants :

-   Extrait en sortie d'adsorbeur, juste en amont de la vanne de contrôle d'extrait :
    Le couple température pression est choisi de la façon suivante: pression variable de 0,9 à 1,4 MPa, température comprise dans l'intervalle de 150 °C à 185 °C,
-   Raffinat en sortie d'adsorbeur : juste en amont de la vanne de contrôle de raffinat :
    Le couple température pression est choisi de la façon suivante: pression variable de 0,9 à 1,4 MPa, température comprise dans l'intervalle de 150 °C à 185 °C, Pour la seconde voie d'analyse (VA2), 3 piquages sont réalisés, chacun d'eux terminé par une vanne et aboutissant en un point commun :

-   Raffinat distillé : localisé au refoulement de la pompe de charge de l'unité d'isomérisation, couple température pression choisi de la façon suivante: température de 120 °C à 160 °C, pression de 1 à 1,6 MPa,
-   Charge : localisé entre le refoulement de la pompe de charge et la vanne de contrôle de charge, couple température pression choisi de la façon suivante: température de 150 °C à 185 °C, pression de 1,6 à 2 MPa,
-   Liquide de recirculation renvoyé du pied de l'adsorbeur A vers la tête de l'adsorbeur B. Le couple pression température est choisi de la façon suivante : température comprise entre

0°C et 80° C pression comprise entre 0,15 à 0,5 MPa relatif.

## EXEMPLES SELON DES MODES DE REALISATION PREFERES DE L'INVENTION

### 1) EXEMPLE DE FONCTIONNEMENT D'UNE VOIE D'ANALYSE (VA1)

- Filtration de particules en suspension (commune aux 2 branches) :

La séparation du paraxylène à partir des coupes aromatiques en C8 s'effectue sur un solide adsorbant généralement une zéolithe de la famille des faujasites.

Ce solide se présente sous forme de billes de taille généralement comprise entre 0,3 et 0,8 mm de diamètre. Ces billes sont elle-même constituées de cristallites.

Lors du chargement initial du solide adsorbant dans l'unité, malgré toutes les précautions prises, des cristallites se détachent des billes, par exemple par attrition ou à cause de la hauteur de chute de la bille.

C'est au moment de la mise en route initiale pendant la période de réglage de l'unité que la concentration en cristallites dans le courant interne de recyclage et dans les effluents (extrait et raffinat) est la plus forte. Il convient donc de filtrer les flux à analyser, pour éviter de fausser la mesure optique et d'encrasser les pièces du dispositif décrit plus loin.

On utilise de préférence des filtres présentant un seuil de coupure compris entre 3 et 15 micromètres qui sont changés régulièrement avec une fréquence variant entre 6 heures au démarrage des adsorbeurs à environ une semaine lorsque les adsorbeurs fonctionnent en régime stabilisé.

On choisit de préférence des crépines réalisées en métal fritté qui présentent l'avantage de pouvoir être réutilisées après nettoyage au bain à ultrason.

- Débit et vitesse de l'échantillon devant l'optrode (branche n°1) :

Ce critère est fixé par 2 considérations :

1°) délai maximum d'acheminement entre le point de prélèvement et le point d'analyse égal à 75 secondes (correspondant à la période de permutation des lits de solide),
2°) temps de pose de chaque mesure dans le spectromètre égal à une seconde.

L'armoire métallique contenant le dispositif d'analyse se trouve positionnée le plus prêt possible du point de prélèvement du flux de recirculation interne. Le diamètre minimal du tuyau soudé sur la canalisation principale pour prélever l'échantillon se situe entre un demi pouce et 15 mm, avec une section de passage d'approximativement 1 cm$^2$, la longueur de ce tuyau est de 5 mètres,

On fait ensuite une réduction de diamètre pour se connecter à la vanne d'entrée de l'armoire métallique contenant l'analyseur, à partir de cette vanne on utilise du tuyau de diamètre 6 mm ou un quart de pouce, avec une section de passage d'approximativement 0,125 cm$^2$. Compte tenu des éléments présents dans l'armoire d'analyse, la longueur équivalente à du tuyau de cette section est de 20 mètres.

Pour respecter le premier des 2 critères de 75 secondes de délai, il vient un débit de 10 cm$^3$/seconde (50 secondes entre la canalisation principale et l'entrée dans l'armoire d'analyse et 25 sec dans le dispositif d'analyse lui-même) soit un débit de 36 l/h.

Le second critère permet de choisir le volume de la cellule à placer en face de l'optrode : pour un temps d'analyse d'une seconde et un débit de 10 cm$^3$/sec un volume d'1 cm$^3$ permet de renouveler 10 fois le volume, ce qui permet la réalisation d'un rinçage parfait.

Choix de la température et de la surface de refroidissement de l'échangeur (3) :

On dispose généralement d'eau de refroidissement de température comprise entre 5 °C et 40 °C selon les différents sites et les différentes saisons. Dans le cas le plus défavorable, (eau de refroidissement à 40°C avec un débit au moins 10 fois plus élevé que celui de l'échantillon à refroidir), température de l'échantillon à analyser d'environ 150 °C à l'entrée de l'armoire d'analyse, température maximale de l'échantillon de 60 °C dans l'enceinte de confinement, il faut une surface d'échange de 0,2 m$^2$ compte tenu d'un coefficient d'échange de 500 W/m$^2$/°C soit 12,5 m de longueur de tuyau de diamètre 6mm, pour un serpentin avec circulation d'eau extérieure.

Pour les sites où la température hivernale peut descendre bien en dessous de 0°C il faut un dispositif de mise hors gel pour éviter que la partie calandre de l'échangeur ne puisse éclater à cause du gel.

Compte tenu de la taille de l'échangeur de refroidissement et des débits et température de l'eau de refroidissement, la température de l'échantillon au point d'analyse est de 15 °C à 60 °C.

Cette température est bien entendu mesurée juste avant le point d'analyse, et prise en compte pour le calcul des compositions de mélange.

Choix de la pression d'épreuve du dispositif d'analyse :

Le calcul de la pression d'épreuve au sein du dispositif d'analyse est effectué par rapport aux valeurs de la pression de refoulement maximale de la pompe de recirculation avec un coefficient de sécurité de 2, la cellule à circulation subit une épreuve hydraulique à 8 MPa.

La pression de service du dispositif est équivalente à celle de la soupape de protection de la capacité contenant le solide adsorbant soit 1,9 MPa.

Une vanne de détente permet de régler la pression en aval entre 0,2 et 0,5 MPa.

Ainsi dimensionnée et opérée, la voie VA1 d'analyse du dispositif selon l'invention permet de réaliser des analyses en lignes par spectroscopie RAMAN, soit du courant de circulation interne renvoyé de l'adsorbeur B vers l'adsorbeur A, soit de l'extrait ou du raffinat issus de l'un ou l'autre des adsorbeurs.

## 2) EXEMPLE DE FONCTIONNEMENT DE LA BRANCHE (BA) D'UNE VOIE D'ANALYSE

En complément de l'analyse Raman, on procède occasionnellement à une série d'analyses par chromatographie en phase vapeur.

Ce prélèvement est automatisé pour une série de N+1 échantillons coordonnée avec les permutations des N lits (N est le nombre de lits de solide adsorbant).

Le superviseur ouvre et referme une électrovanne qui envoie de l'air comprimé vers la vanne pneumatique pour diriger l'échantillon vers un flacon de prélèvement. Une séquence typique d'échantillonnage est synchronisée avec la permutation des lits d'adsorbant :

T=0 permutation du 1° lit, le flux circule dans la branche n° 2 du cabinet d'analyse Raman,
T = 5 secondes ouverture pendant 5 sec. (abréviation de seconde) vers l'échantillonnage extérieur : rinçage des lignes,
T= 10 sec fermeture pendant 5 sec. de l'échantillonnage, le flux circule dans la branche n° 2 du cabinet d'analyse Raman,
T= 15 sec ouverture pendant 10 sec. vers l'échantillonnage extérieur : prélèvement de 100 cm3 du flacon n°1,
T = 25 sec. fermeture, le flux circule dans la branche n° 2 du cabinet d'analyse Raman,
T=75 sec. permutation du 2° lit, le flux circule dans la branche n° 2 du cabinet d'analyse Raman,

T = 80 sec. ouverture pendant 5 sec. vers l'échantillonnage extérieur : rinçage des lignes,

T= 85 sec. fermeture pendant 5 sec. de l'échantillonnage,

T = 90 sec. ouverture pendant 10 sec. vers l'échantillonnage extérieur : prélèvement de 100 cm3 du flacon n°2,

T = 100 sec. fermeture, le flux circule dans la branche n° 2 du cabinet d'analyse Raman

Ce processus est répété successivement pour chacun des lits de la boucle. S'il y en a 24 on arrive à:

T=1725 sec. permutation du 24° lit, le flux circule dans la branche n° 2 du cabinet d'analyse Raman,

T = 1730 sec. ouverture pendant 5 sec. vers l'échantillonnage extérieur : rinçage des lignes,

T= 1735 sec. fermeture pendant 5 sec. de l'échantillonnage,

T=1740 sec. ouverture pendant 10 sec. vers l'échantillonnage extérieur : prélèvement de 100 cm3 du flacon n°24,

T = 1750 sec. fermeture, le flux circule dans la branche n° 2 du cabinet d'analyse Raman.

De manière à vérifier que l'échantillonnage s'est déroulé de manière satisfaisante, on prélève une seconde fois un échantillon du premier lit (flacon N° 25 s'il y a 24 lits), les 2 analyses devant être identiques.

Dés que cet échantillonnage est terminé, on rétablit les conditions normales de circulation de la voie d'analyse (passage par la branche n°1 du circuit).

Lorsque tous les résultats d'analyse par chromatographie des 25 échantillons sont disponibles (généralement environ 24 heures après les prises d'échantillons), en plus de la mesures des constituants présents sous forme de traces (constituants non aromatiques à 8 et 9 atomes de carbone, et constituants aromatiques à 9 et 10 atomes de carbone) il est possible de comparer pour le toluène, les trois xylènes (ortho, meta, para), l'ethylbenzène et le paradiethylbenzène, les valeurs obtenues par spectroscopie Raman juste avant et juste après cet échantillonnage. Ceci permet de détecter une éventuelle dérive des résultats rendant nécessaire une nouvelle calibration du spectromètre Raman.

Les analyses en ligne par spectroscopie Raman ainsi calibrées permettent, en utilisant par exemple tout ou partie de la méthode d'analyse RAMAN décrite dans le brevet US 8,194,245, de suivre en continue le fonctionnement des adsorbeurs au moyen du dispositif selon l'invention et de détecter tout disfonctionnement avec un très faible décalage dans le temps.

**Revendications**

**1.** Unité de séparation des xylènes en lit mobile simulé, dite unité LMS, comportant deux adsorbeurs (A) et

(B) connectés en série, c'est à dire avec le courant de circulation issu du pied de l'adsorbeur (A) renvoyé en tête de l'adsorbeur (B) et avec le courant de circulation issu du pied de l'adsorbeur (B) renvoyé en tête de l'adsorbeur (A), ladite unité comportant une colonne à distiller (C) alimentée par le raffinat (RAF B) issu de l'adsorbeur (B) et ladite unité comportant un dispositif de mesure de concentration par analyse d'un spectre Raman, ledit dispositif de mesure comportant un spectromètre Raman pour la mesure des concentrations en différents points de prélèvement, **caractérisé en ce que** ledit dispositif de mesure étant constitué de deux voies identiques et indépendantes (VA1, VA2), dites voie d'analyse 1 (VA1) et voie d'analyse 2 (VA2), chaque voie d'analyse (VA1, VA2) possédant deux branches (BP, BA), l'une dite branche principale (BP) étant configurée pour fonctionner la majeure partie du temps, l'autre dite branche annexe (BA) étant configurée pour fonctionner une mineure partie du temps, et les points de prélèvements étant définis de la manière suivante :

- pour la voie d'analyse 1 (VA1) connectée au courant de circulation interne allant de l'adsorbeur (B) à l'adsorbeur (A),

a) courant de circulation interne renvoyé de l'adsorbeur (B) vers l'adsorbeur (A),
b) extrait (EX A, EX B) issu de l'un ou l'autre des adsorbeurs (A, B),
c) raffinat (RAF A, RAF B) issu de l'un ou l'autre des adsorbeurs (A, B),

- pour la voie d'analyse 2 (VA2) connectée au raffinat distillé (RAF DIST) issu de la colonne de distillation (C),

d) raffinat distillé (RAF DIST), en sortie de la colonne de distillation (C),
e) courant de circulation interne de l'adsorbeur (A) vers l'adsorbeur (B),
f) charge de l'unité en entrée de l'un ou l'autre des adsorbeurs (A, B),

lesdites deux branches (BP, BA) étant distinctes et configurées pour fonctionner de manière exclusif l'une de l'autre, la

branche 1 (BP), dite "principale" (BP) étant utilisée pour l'analyse Raman et la branche 2 (BA) dite "annexe" (BA) étant utilisée pour le prélèvement d'échantillon,

et la branche 1 (BP) de chaque voie d'analyse (VA1, VA2) comportant les éléments principaux suivants pris dans le sens du flux à analyser:

- une vanne d'arrêt (6) permettant de forcer la circulation dans la branche 2 (BA) dans certaines situations,
- un thermocouple (7) permettant une prise de température de l'échantillon à analyser,
- une cellule d'analyse (8) contenant une optrode (10), recevant un signal laser d'entrée et envoyant un signal de sortie vers le spectromètre Raman, le volume interne de ladite cellule d'analyse (8) étant de l'ordre de 1 cm$^3$,
- une seconde vanne d'arrêt (9) pour isoler la cellule d'analyse (8) en cas de maintenance.

2. Unité de séparation des xylènes en lit mobile simulé, dite unité LMS, selon la revendication 1, dans lequel la cellule d'analyse (8) et l'optrode (10) sont contenues dans la même cellule de confinement.

3. Unité de séparation des xylènes en lit mobile simulé, dite unité LMS, selon la revendication 1, comprenant une fibre optique d'entrée dans lequel l'optrode (10) est reliée à ladite fibre optique d'entrée amenant un signal laser de longueur d'onde comprise entre 400 et 1080 nm, et préférentiellement comprise entre 520 et 785 nm.

4. Unité de séparation des xylènes en lit mobile simulé, dite unité LMS, selon la revendication 1, dans laquelle en cas de maintenance sur la voie d'analyse VA1, la voie d'analyse VA2 est configurée pour être connectée au courant de circulation interne de l'adsorbeur (A) vers l'adsorbeur (B).

5. Unité de séparation des xylènes en lit mobile simulé, dite unité LMS, selon la revendication 1, dans laquelle le volume total du circuit entre le point de prélèvement et le point de mesure est tel que le temps de circulation entre lesdits points est compris entre 1 et 75 secondes, la vitesse linéaire dans la cellule d'analyse (8) étant comprise entre 0,02 et 0,25 m/s.

6. Méthode d'analyse des concentrations sur une unité de séparation des xylènes en lit mobile simulé, dite unité LMS, selon l'une quelconque des revendications 1 à 5, **caractérisé par le fait que** les éléments du dispositif sont calculés pour assurer la combinaison de conditions opératoires suivantes au niveau de la cellule d'analyse (8):

1) échantillon substantiellement exempt de particules solides en suspension (en contenant moins de 10 ppb),
2) échantillon dont la température est comprise entre 20 °C et 60 °C,
3) échantillon dont la pression est comprise entre 0,2 et 0,5 MPa,
4) vitesse linéaire comprise entre 0,02 et 0,25 m/sec, de préférence entre 0,05 et 0,2 m/sec, et de manière très préférée entre 0,08 et 0,12 m/sec.

7. Méthode d'analyse des concentrations sur une unité de séparation des xylènes en lit mobile simulé, dite unité LMS, selon l'une quelconque des revendications 1 à 5, dans laquelle en cas de fonctionnement dégradé de l'unité en lit mobile simulé on peut déconnecter la ligne d'échantillonnage de la première voie d'analyse pour la connecter sur l'un des points suivants:

- l'extrait en sortie d'adsorbeur, juste en amont de la vanne de contrôle d'extrait :
le couple température pression étant choisi de la façon suivante: pression variable de 0,9 à 1,4 MPa, température dans l'intervalle de 150 °C et 185 °C.
- le raffinat en sortie d'adsorbeur : juste en amont de la vanne de contrôle de raffinat :
le couple température pression étant choisi de la façon suivante: pression variable de 0,9 à 1,4 MPa, température comprise entre 150 °C et 185 °C.

8. Méthode d'analyse des concentrations sur une unité de séparation des xylènes en lit mobile simulé, dite unité LMS, selon l'une quelconque des revendications 1 à 5, dans laquelle la seconde voie d'analyse (VA2) s'applique à l'un des points de prélèvement suivants :

- Raffinat distillé (RAF DIST): localisé au refoulement de la pompe de charge de l'unité d'isomérisation, température comprise entre 120°C à 160 °C, pression comprise entre 1 à 1,6 MPa,
- Charge : localisé entre le refoulement de la pompe de charge et la vanne de contrôle de charge, température comprise entre 150°C et 185 °C, pression comprise entre 1,6 à 2 MPa,
- Liquide de recirculation renvoyé du pied de l'adsorbeur (A) vers la tête de l'adsorbeur

(B), température comprise entre 0 °C et 80 °C, pression comprise entre 0,15 à 0,5 MPa relatif.

**Patentansprüche**

1. Einheit zur Trennung von Xylolen im simulierten Fließbett, welche als LMS Einheit bezeichnet wird, umfassend zwei Adsorber (A) und (B), die in Serie verbunden sind, das heißt, dass der Zirkulationsstrom vom Fuß des Adsorbers (A) zum Kopf des Adsorbers (B) zurückgeführt wird, und dass der Zirkulationsstrom vom Fuß des Adsorbers (B) zum Kopf des Adsorbers (A) zurückgeführt wird, wobei die Einheit eine Destillationssäule (C) umfasst, die mit dem Raffinat (RAF B) aus dem Adsorber (B) versorgt wird, und wobei die Einheit eine Vorrichtung zur Messung der Konzentration durch eine Raman-Spektralanalyse umfasst, wobei die Messvorrichtung ein Raman-Spektrometer zur Messung der Konzentrationen an verschiedenen Entnahmepunkten umfasst, **dadurch gekennzeichnet, dass** die Messvorrichtung aus zwei identischen und unabhängigen Wegen (VA1, VA2) besteht, die als Analyseweg 1 (VA1) und Analyseweg 2 (VA2) bezeichnet werden, wobei jeder Analyseweg (VA1, VA2) zwei Verzweigungen (BP, BA) aufweist, wobei die eine, die als Hauptverzweigung (BP) bezeichnet wird, ausgelegt ist, um den Großteil der Zeit betrieben zu werden, wobei die andere, die als Nebenverzweigung (BA) bezeichnet wird, ausgelegt ist, um einen geringen Teil der Zeit betrieben zu werden, und wobei die Entnahmepunkte auf folgende Weise definiert sind:

- für den Analyseweg 1 (VA1), welcher mit dem internen Zirkulationsstrom verbunden ist, der vom Adsorber (B) zum Adsorber (A) verläuft:

a) interner Zirkulationsstrom, der vom Adsorber (B) zum Adsorber (A) zurückgeschickt wird,
b) Extrakt (EX A, EX B) aus dem einen oder dem anderen der Adsorber (A, B),
c) Raffinat (RAF A, RAF B) aus dem einen oder dem anderen der Adsorber (A, B),

- für den Analyseweg 2 (VA2), der mit dem destillierten Raffinat (RAF DIST) aus der Destillationssäule (C) verbunden ist:

d) destilliertes Raffinat (RAF DIST) am Ausgang der Destillationssäule (C),
e) interner Zirkulationsstrom von dem Adsorber (A) zum Adsorber (B),
f) Charge der Einheit am Eingang des einen oder des anderen der Adsorber (A, B),

wobei die beiden Verzweigungen (BP, BA) verschieden sind und ausgelegt sind, um einander ausschließend betrieben zu werden, wobei die Verzweigung 1 (BP), die als "Haupt"verzweigung (BP) bezeichnet wird, für die Raman-Analyse verwendet wird, und wobei die Verzweigung 2 (BA), die als "Neben"verzweigung (BA) bezeichnet wird, für die Probenentnahme verwendet wird, und wobei die Verzweigung 1 (BP) jedes Analysewegs (VA1, VA2) die folgenden Hauptelemente, gesehen in der zu analysierenden Flussrichtung, umfasst:

- ein Sperrventil (6), das es ermöglicht, die Zirkulation in der Verzweigung 2 (BA) in bestimmten Situationen zu erzwingen,
- ein Thermoelement (7), das eine Temperaturabnahme der zu analysierenden Probe ermöglicht,
- eine Analysezelle (8), welche eine Optrode (10) enthält, die ein Laser-Eingangssignal empfängt und ein Ausgangssignal an das Raman-Spektrometer sendet, wobei das interne Volumen der Analysezelle (8) in der Größenordnung von 1 cm$^3$ liegt,
- ein zweites Sperrventil (9) zur Isolation der Analysezelle (8) im Fall einer Wartung.

2. Einheit zur Trennung von Xylolen im simulierten Fließbett, welche als LMS Einheit bezeichnet wird, nach Anspruch 1, wobei die Analysezelle (8) und die Optrode (10) in derselben abgeschlossenen Zelle enthalten sind.

3. Einheit zur Trennung von Xylolen im simulierten Fließbett, welche als LMS Einheit bezeichnet wird, nach Anspruch 1, umfassend eine optische Eingangsfaser, wobei die Optrode (10) mit der optischen Eingangsfaser verbunden ist, die ein Laser-Wellenlängensignal zwischen 400 und 1080 nm, und vorzugsweise zwischen 520 und 785 nm, führt.

4. Einheit zur Trennung von Xylolen im simulierten Fließbett, welche als LMS Einheit bezeichnet wird, nach Anspruch 1, umfassend eine optische Eingangsfaser, wobei, im Fall einer Wartung auf dem Analyseweg VA1, der Analyseweg VA2 ausgelegt ist, um mit dem internen Zirkulationsstrom vom Adsorber (A) zum Adsorber (B) verbunden zu werden.

5. Einheit zur Trennung von Xylolen im simulierten Fließbett, welche als LMS Einheit bezeichnet wird, nach Anspruch 1, wobei das Gesamtvolumen des Kreislaufs zwischen dem Entnahmepunkt und dem Messpunkt derart ist, dass die Zirkulationszeit zwischen den Punkten zwischen 1 und 75 Sekunden liegt, wobei die lineare Geschwindigkeit in der Analysezelle (8) zwischen 0,02 und 0,25 m/s liegt.

6. Verfahren zur Analyse von Konzentrationen in einer Einheit zur Trennung von Xylolen im simulierten Fließbett, die als LMS Einheit bezeichnet wird, nach einem der Ansprüche 1 bis 5, **dadurch gekennzeichnet, dass** die Elemente der Vorrichtung berechnet werden, um die Kombination der folgenden Betriebsbedingungen auf der Höhe der Analysezelle (8) sicherzustellen:

1) Probe im Wesentlichen frei von festen Partikeln in Suspension (enthaltend weniger als 10 ppb davon),
2) Probe, deren Temperatur zwischen 20 °C und 60 °C liegt,
3) Probe, deren Druck zwischen 0,2 und 0,5 MPa liegt,
4) lineare Geschwindigkeit zwischen 0,02 und 0,25 m/s, vorzugsweise zwischen 0,05 und 0,2 m/s, und sehr bevorzugt zwischen 0,08 und 0,12 m/s.

7. Verfahren zur Analyse von Konzentrationen in einer Einheit zur Trennung von Xylolen im simulierten Fließbett, die als LMS Einheit bezeichnet wird, nach einem der Ansprüche 1 bis 5, wobei im Fall eines verschlechterten Betriebs der Einheit im simulierten Fließbett die Probenentnahmeleitung von dem ersten Analyseweg getrennt werden kann, um sie mit einem der folgenden Punkte zu verbinden:

- Extrakt am Ausgang des Adsorbers, knapp stromaufwärts von dem Extraktsteuerventil: wobei das Paar Temperatur-Druck auf folgende Weise ausgewählt wird: Druck variabel von 0,9 bis 1,4 MPa, Temperatur im Intervall von 150 °C und 185 °C,
- Raffinat am Ausgang des Absorbers: knapp stromaufwärts von dem Raffinatsteuerventil: wobei das Paar Temperatur-Druck auf folgende Weise ausgewählt wird: Druck variabel von 0,9 bis 1,4 MPa, Temperatur zwischen 150 °C und 185 °C.

8. Verfahren zur Analyse von Konzentrationen in einer Einheit zur Trennung von Xylolen im simulierten Fließbett, die als LMS Einheit bezeichnet wird, nach einem der Ansprüche 1 bis 5, wobei der zweite Analyseweg (VA2) für einen der folgenden Entnahmepunkte gilt:

- destilliertes Raffinat (RAF DIST): lokalisiert am Auslass der Ladungspumpe der Isomerisierungseinheit, Temperatur zwischen 120 °C und 160 °C, Druck zwischen 1 bis 1,6 MPa,
- Charge: lokalisiert zwischen dem Auslass der Ladungspumpe und dem Chargensteuerventil, Temperatur zwischen 150°C und 185 °C, Druck zwischen 1,6 bis 2 MPa,
- Rezirkulationsflüssigkeit, die vom Fuß des Absorbers (A) zum Kopf des Absorbers (B) zurückgeschickt wird, Temperatur zwischen 0 °C und 80 °C, Druck zwischen 0,15 bis 0,5 MPa relativ.

**Claims**

1. Simulated moving bed xylenes separation unit, called an SMB unit, comprising two adsorbers (A) and (B) connected in series, i.e. with the circulation current originating from the bottom of adsorber (A) sent to the top of adsorber (B) and with the circulation current originating from the bottom of adsorber (B) sent to the top of adsorber (A), said unit comprising a distillation column (C) fed by the raffinate (RAF B) originating from the adsorber (B) and said unit comprising a device for measuring concentration by analyzing a Raman spectrum, said measuring device comprising a Raman spectrometer for measuring concentrations at different sampling points, **characterized in that** said measuring device is constituted by two identical and independent routes (AR1, AR2), called analysis route 1 (AR1) and analysis route 2 (AR2), each analysis route (AR1, AR2) having two branches (MP, SB), one called main branch (MP) being configured for operating for a majority of the time, the other called the subsidiary branch (SB) being configured for operating for a minority of the time, and the sampling points being defined as follows:

- for analysis route 1 (AR1) connected to the internal circulation current going from adsorber (B) to adsorber (A),

a) internal circulation current sent from adsorber (B) to adsorber (A),
b) extract (EX A, EX B) originating from one or other of the adsorbers (A, B),
c) raffinate (RAF A, RAF B) originating from one or other of the adsorbers (A, B),

- for analysis route 2 (AR2) connected to the distilled raffinate (RAF DIST) originating from distillation column (C),

d) distilled raffinate (RAF DIST) leaving distillation column (C),
e) internal circulation current from adsorber (A) to adsorber (B),
f) feed of the unit at the inlet of one or other of the adsorbers (A, B), said two branches (MP, SB) being distinct and configured for operating with mutually exclusive operation, branch 1 (MP), called the "main branch" (MB) being used for the Raman analysis and branch 2 (SB), called the "subsidiary branch" (SB) being used for sample collection, and branch 1 (MP) of each analysis route (AR1, AR2) comprising the following main components taken in the direction of the stream to be analyzed:

- a shut-off valve (6) making it possible to force the circulation into branch 2 (SB) in certain situations,
- a thermocouple (7) making it possible to take the temperature of the sample to be analyzed,
- an analysis cell (8) containing an optrode (10), receiving an input laser signal and sending an output signal to the Raman spectrometer, the internal volume of said analysis cell (8) being of the order of 1 cm$^3$,
- a second shut-off valve (9) for isolating the analysis cell (8) in the event of maintenance.

2. Simulated moving bed xylenes separation unit, called an SMB unit, according to claim 1, in which the analysis cell (8) and the optrode (10) are contained in the same containment cell.

3. Simulated moving bed xylenes separation unit, called an SMB unit, according to claim 1, comprising a input optical fiber in which the optrode (10) is linked to said input optical fibre providing a laser signal at a wavelength comprised between 400 and 1080 nm, and preferentially comprised between 520 and 785 nm.

4. Simulated moving bed xylenes separation unit, called an SMB unit, according to claim 1, in which in the event of maintenance on the analysis route AR1, the analysis route AR2 is configured for being connected to the internal circulation current from adsorber (A) to adsorber (B).

5. Simulated moving bed xylenes separation unit, called an SMB unit, according to claim 1, in which the total volume of the circuit between the sampling point and the measuring point is such that the circulation time between said points is comprised between 1 and 75 seconds, the linear speed in the analysis cell (8) being comprised between 0.02 and 0.25 m/s.

6. Method for analyzing concentrations on a simulated moving bed xylenes separation unit, called an SMB unit, according to any one of claims 1 to 5, **characterized by** the fact that the components of the device are calculated in order to ensure the following combination of operating conditions in the analysis cell (8):

    1) sample substantially free of solid particles in suspension (containing less than 10 ppb),
    2) sample the temperature of which is comprised between 20 °C and 60 °C,
    3) sample the pressure of which is comprised between 0.2 and 0.5 MPa,
    4) linear speed comprised between 0.02 and 0.25 m/sec, preferably between 0.05 and 0.2 m/sec, and very preferably between 0.08 and 0.12 m/sec.

7. Method for the analysis of concentrations on a simulated moving bed xylenes separation unit, called an SMB unit, according to any one of claims 1 to 5, in which in the event of the simulated moving bed unit operating in degraded mode, the sampling line may be disconnected from the first analysis route in order to connect it at one of the following points:

    - The extract leaving the adsorber, just upstream of the extract control valve:
    the pressure-temperature pair being chosen as follows: pressure variable from 0.9 to 1.4 MPa, temperature in the range from 150 °C to 185 °C.
    - The raffinate leaving the adsorber: just upstream of the raffinate control valve:
    the pressure-temperature pair being chosen as follows: pressure variable from 0.9 to 1.4 MPa, temperature comprised between 150 °C and 185 °C.

8. Method for the analysis of concentrations on a simulated moving bed xylenes separation unit, called an SMB unit, according to any one of claims 1 to 5, in which the second analysis route (AR2) is applied to one of the following sampling points:

    - Distilled raffinate (RAF DIST): located at the discharge of the feed pump of the isomerization unit, temperature comprised between 120 °C to 160 °C, pressure comprised between 1 to 1.6 MPa,
    - Feed: located between the discharge of the feed pump and the feed control valve, temperature comprised between 150 °C and 185 °C, pressure comprised between 1.6 to 2 MPa,
    - Recirculation liquid sent from the bottom of adsorber (A) to the top of adsorber (B), temperature comprised between 0° and 80 °C, pressure comprised between 0.15 to 0.5 MPa relative.

FIGURE 1

EP 2 813 840 B1

FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5569808 A
- US 8194245 B
- US 7116414 B
- US 7548310 B
- US 20130053610 A1